# EUROPÄISCHE PATENTSCHRIFT

(11) **EP 3 411 087 B1**
(45) Veröffentlichungstag und Bekanntmachung des Hinweises auf die Patenterteilung: **19.06.2019**
(21) Anmeldenummer: 17712946.7
(22) Anmeldetag: 20.03.2017
(51) Int. Cl.: B60H 3/06, A61L 9/20, B01D 53/00, B01D 53/88, B01J 35/00, B01J 21/06

(54) **LUFTREINIGUNGSVORRICHTUNG**
AIR PURIFICATION DEVICE
DISPOSITIF D'ÉPURATION DE L'AIR

(30) Priorität: 22.03.2016 DE 102016105276
(43) Veröffentlichungstag der Anmeldung: 12.12.2018
(73) Patentinhaber: Dr. Schneider Kunststoffwerke GmbH, 96317 Kronach (DE)
(72) Erfinder: FIEDLER, Jochen, 96332 Pressig (DE); ENDRES, Gerhard, 95336 Mainleus (DE)
(86) Internationale Anmeldenummer: PCT/EP2017/056537
(87) Internationale Veröffentlichungsnummer: WO 2017/162577

(56) Entgegenhaltungen:
- EP-A1- 2 181 720
- WO-A1-2016/105224
- JP-A- H11 157 332
- US-A- 5 919 422

## Beschreibung

Die vorliegende Einrichtung dient zur Reinigung von Luft, insbesondere zur Reinigung von Luft in Kraftfahrzeuginnenräumen.

Aus dem Stand der Technik sind verschiedene Vorrichtungen zur Luftreinigung in Kraftfahrzeugen bekannt. Diese sind zumeist derart ausgebildet und angeordnet, dass Luft kontinuierlich aus dem Fahrzeuginnenraum abgesaugt, über einen Filter geführt und dann dem Fahrzeuginnenraum wieder zugeführt wird.

So ist aus der DE 10 2014 012 870 A1 ein Luftreiniger bekannt. Dieser Luftreiniger verwendet ultraviolette Strahlen zur Luftreinigung. Der Luftreiniger weist ein Gehäuse mit einem Gehäuseeinlass und einem -auslass, ein Gebläse, das im inneren des Gehäuses angrenzend an den Lufteinlass angeordnet ist, eine ultraviolette Leuchtdiodeneinheit und eine Filtereinheit auf. Die Filtereinheit ist im Inneren des Gehäuses über dem Gebläse entlang eines Strömungsweges der Luft angeordnet. Es ist außerdem eine Strömungsanordnung vorhanden, die im Inneren des Gehäuses zwischen dem Gebläse und der Filtereinheit angeordnet ist. Dabei steuert die Strömungssteueranordnung den Luftstrom entlang des Strömungswegs der Luft zwischen dem Auslass des Gebläses und der Filtereinheit. Zur Reinigung der Luft kommen hierbei fotokatalytische, ultraviolette Leuchtdioden zum Einsatz.

Aus der DE 20 2007 019 288 U1 ist ein System zur Luftreinigung unter Verwendung von Ozon und einem keramischen, porösen Katalysator offenbart. Das System weist ein Gehäuse, mindestens einen Einlass und einen Auslass, mindestens eine Photonenquelle, einen keramischen Kern und ein Fluidstromerzeugungsgerät auf, wobei die Photonenquelle stromaufwärts von dem keramischen Kern angeordnet ist.

Aus der US 5 919 422 A ist ein Photokatalysator zum Deodorieren, Reinigen, Sterilisieren und zur Reinigung von Wasser oder Luft gemäß Oberbegriff des Anspruchs 1 offenbart. Dieser besitzt ein Substrat, auf dem ein Titanoxidfilm angeordnet ist. Im Weiteren ist eine Leuchtdiode, die neben dem Titanoxidfilm angeordnet ist, vorhanden, welche ultraviolettes Licht mit einer Wellenlänge von 360 bis 400 Nanometern ermittieren und auf den Titandioxidfilm strahlt.

Aus der EP 2 181 720 A1 ist eine Vorrichtung mit einer UVA-Strahlenquelle offenbart, die eine Oberfläche eines Trägers beleuchtet, der einen Photokatalysator auf Titanoxidbasis besitzt. Der Träger ist mit einer inneren Schicht versehen, die aus einer metallischen Kupferplatte besteht. Der Träger ist mit einer Kohlenstoffinnenschicht und zwei äußeren Schichten, einschließlich des Photokatalysators, versehen. Es ist eine Luftzirkulationseinheit zur Erzeugung einer Bewegung der Luft entlang des Trägers vorgesehen, sodass die erzeugte Luftströmung durch und über den Träger hindurchtritt. Die feste metallische Verbindung wird aus bakteriostatischen oder antimikotischen oder keimtötenden Metallen erzeugt.

Aus der EP 0 707 989 A1 ist eine Vorrichtung zur Reinigung von Luft bekannt. Eine Reinigung erfolgt in der Weise, dass Außenluft über ein Gehäuse und ein darin angeordnetes Gebläse angesaugt, in mehreren Reinigungsstufen gereinigt und dann dem Innenraum des Fahrzeugs zugeführt wird. Das Gebläse bzw. der Antrieb des Gebläses wird über Solarzellen, die am Fahrzeug angeordnet sind und als Energiequellen dienen, gespeist.

Nachteilig bei dem vorgenannten Stand der Technik ist, dass die Reinigungswirkung und insbesondere die Beseitigung von Schadpartikeln oder Geruchspartikeln aus der zu reinigenden Luft nicht ausreichend und nicht über einen längeren Zeitraum ohne Wartungseingriffe möglich sind. Dies ist zum einen dadurch bedingt, dass zumeist ein mechanischer Filter verwendet wird, der nach relativ kurzer Zeit bereits eine solche Menge an Partikeln aufgenommen hat, dass er getauscht werden muss, um die ursprüngliche Reinigungsleistung beizubehalten. Erfolgt kein regelmäßiger Austausch, trägt ein solcher Filter mehr zur Verunreinigung der Luft bei, als dass er diese reinigt. Es dürfte den meisten Benutzern von Klimaanlagen bekannt sein, dass ein verschmutzter Filter erheblich zur Geruchsbelästigung in einem Kraftfahrzeug beiträgt.

Weiterhin ist nachteilig, dass ein Filterwechsel mit relativ hohen Kosten verbunden ist, da ein solcher Wechsel zumeist nur in einer Fachwerkstatt vorgenommen werden kann.

Im Weiteren ist zu berücksichtigen, dass vor allem neue Fahrzeuge über einen bestimmten Zeitraum flüchtige organische Verbindungen (VOC) und andere gesundheitsschädlich Substanzen abgeben. Um diese möglichst umgehend und ohne Belastung für die Insassen eines Fahrzeugs zu beseitigen, sind aus dem Stand der Technik bisher keine Systeme bekannt. Zwar sind im Stand der Technik Filtersysteme, wie eingangs beschrieben, vorhanden, welche auch solche Verbindungen herausfiltern können, jedoch ist der Zeitraum, über welchen sich diese Verbindung im Fahrzeug halten, verhältnismäßig lang.

Es ist daher Aufgabe der vorliegenden Erfindung, die Nachteile des Standes der Technik zu beheben und eine Luftreinigungsvorrichtung anzugeben, welche flüchtige organische Stoffe und gesundheitsschädlich Substanzen sowie andere Luftverunreinigungen gezielt in der Luft im Fahrzeuginnenraum reduziert und zusätzlich auch unangenehme Gerüche vermeidet und beseitigt.

Diese Aufgabe wird durch eine Luftreinigungssvorrichtung mit den in Anspruch 1 angegebenen technischen Merkmalen gelöst. Vorteilhafte Weiterbildungen sind in den Unteransprüchen, der weiteren Beschreibung und insbesondere der Beschreibung anhand konkreter Ausführungsbeispiele angegeben.

Die vorliegende Erfindung betrifft eine Luftreinigungseinheit mit einem Gehäuse, das mindestens eine Eingangsöffnung zur Zuführung eines Luftstroms und mindestens eine Ausgangsöffnung zur Ausleitung des über die Eingangsöffnung zugeführten Luftstroms besitzt. Im Gehäuse sind mindestens eine Luftreinigungseinheit und mindestens eine Beleuchtungseinheit angeordnet, wobei die mindestens eine Luftreinigungseinheit und die mindestens eine Beleuchtungseinheit einander gegenüberliegend im Gehäuse angeordnet sind. Die mindestens eine Luftreinigungseinheit besitzt mindestens einen photokatalytisch wirksamen Oberflächenbereich. Der Luftstrom wird im Gehäuse zumindest partiell entlang des mindestens einen photokatalytisch wirksamen Oberflächenbereichs der mindestens einen Luftreinigungseinheit geführt, wobei der mindestens eine photokatalytisch wirksame Oberflächenbereich der mindestens einen Luftreinigungseinheit von der mindestens einen Beleuchtungseinheit mit Licht anstrahlbar ist und der mindestens eine photokatalytisch wirksame Oberflächenbereich zumindest teilweise mit Titandioxid - TiO2 - beschichtet oder mit Titandioxidionen - TiO2-Ionen - dotiert ist.

Der Untergrund der Erfindung besteht die TiO2-Schicht aus einer anorganischen Schicht, die gleichzeitig reflektierende Wirkung hat. Dadurch wird verhindert, dass das aktive TiO2 organische Klebeschichten angreift und ggf. auflöst, wobei gleichzeitig die Energie des Lichts effizienter genutzt und verteilt wird.

Es ist vorgesehen, dass die mindestens eine Luftreinigungseinheit an einer der Innenwände des Gehäuses angeordnet ist oder dass die mindestens eine Luftreinigungseinheit in der Mitte des Gehäuses dieses in einer Ebene durchlaufend, angeordnet ist. Durch die Anordnung der mindestens einen Luftreinigungseinheit an einer der Innenwände des Gehäuses kann eine gute Befestigung erfolgen, zugleich kann der Luftstrom im Gehäuse so gelenkt werden, dass er optimal auf die mindestens eine Luftreinigungseinheit abgestimmt ist. Bei einer Anordnung in der Mitte des Gehäuses kann die mindestens eine Luftreinigungseinheit direkt vom in das Gehäuse einströmenden Luftstrom umgeben werden und es wird eine gute Reinigungsleistung erzielt.

Weiterhin ist vorgesehen, dass die mindestens eine Beleuchtungseinheit im Gehäuse der mindestens einen Luftreinigungseinheit gegenüberliegend auf einer der Innenwände des Gehäuses oder in der Mitte des Gehäuses, dieses in einer Ebene durchlaufend, angeordnet ist. Die Beleuchtungseinheit ist zwingend notwendig, da nur durch auf die mindestens eine Luftreinigungseinheit bzw. deren photokatalytisch wirksame Oberflächenbereichen auftreffendes Licht, vorzugsweise UV-Licht, der photokatalytische Reinigungsprozess erfolgt. Daher ist durch diese Anordnung der mindestens einen Beleuchtungseinheit gegenüber der mindestens einen Luftreinigungseinheit eine gute Beleuchtung und damit Zuführung von UV-Licht möglich. Die Reinigungswirkung ist auch mit Deep Blue (sichtbares Licht) erzeugbar und die Erfindung damit auch mit Deep Blue (sichtbares Licht) möglich; es muss aber dann ein dotiertes TiO2 verwendet werden.

Außerdem ist vorgesehen, dass die mindestens eine Beleuchtungseinheit im Gehäuse der mindestens einen Luftreinigungseinheit gegenüberliegend derart angeordnet ist, dass das von der mindestens einen Beleuchtungseinheit emittierte Licht nahezu vollständig auf die mindestens eine Luftreinigungseinheit auftrifft.

In einer vorteilhaften Ausgestaltung der Erfindung nach Patentanspruch 2 ist vorgesehen, dass vor der mindestens einen Beleuchtungseinheit Linsen angeordnet sind, die das von der mindestens einen Beleuchtungseinheit emittierte Licht auf die mindestens einen Luftreinigungseinheit fokussieren oder hinter und/oder neben der mindestens einen Beleuchtungseinheit Lichtreflektoren oder lichtreflektierende Bereiche angeordnet sind, welche das von der mindestens einen Beleuchtungseinheit emittierte Licht in Richtung der mindestens einen Luftreinigungseinheit reflektieren. Durch die Anordnung der Linsen wird eine besonders gute Fokussierung des emittierten Lichts auf die mindestens einen Luftreinigungseinheit erreicht. Durch die Anordnung von Lichtreflektoren oder lichtreflektierende Bereiche wird erreicht, dass auch Licht, das nicht direkt auf die mindestens eine Luftreinigungseinheit gerichtet wird, dennoch zumindest teilweise zur Luftreinigungseinheit geleitet wird.

In einer vorteilhaften Ausgestaltung der Erfindung nach Patentanspruch 3 ist vorgesehen, dass die Innenwände des Gehäuses (2, 22) weiß lackiert oder mit einem lichtreflektierenden Material beschichtet sind. Dadurch wird erreicht, dass das von der mindestens einen Beleuchtungseinheit emittierte Licht nicht vom Gehäuse absorbiert wird, sondern reflektiert und damit zusätzlich auf die bzw. in Richtung der mindestens einen Luftreinigungseinheit geleitet wird.

In einer vorteilhaften Ausgestaltung der Erfindung nach Patentanspruch 4 ist vorgesehen, dass die mindestens eine Beleuchtungseinheit aus mindestens einer Leuchtdiode besteht, die UV-Licht emittiert. Je nach Dotierung des Photokatalysators kann eine Aktivierung auch mit sichtbarem Licht erfolgen.

In einer vorteilhaften Ausgestaltung der Erfindung nach Patentanspruch 5 ist vorgesehen, dass die mindestens eine Luftreinigungseinheit sich nahezu über die gesamte Fläche des Gehäusebodens und/oder des Gehäusedeckels des Gehäuses erstreckt und am Gehäuseboden und/oder Gehäusedeckel fixierbar ist. Damit kann eine große Fläche zur Luftreinigung verwendet werden und die mindestens eine Luftreinigungseinheit kann gut im Gehäuse fixiert werden.

Es ist erfindungsgemäß vorgesehen, dass die mindestens eine Luftreinigungseinheit auf der mindestens den einen katalytisch wirksamen Oberflächenbereich aufweisenden Seite mit einer kegel-, falten- zylinder-, kegelstumpf-, pyramidenstumpf-, kugel- oder halbkugelgeometrischen Form, oder igelförmig, ausgestaltet ist. Durch diese Formgebung kann die für die Luftreinigung wirksame Oberfläche der mindestens einen Luftreinigungseinheit vergrößert werden. Die konkrete Formgestaltung der Oberfläche führt aber nur zu einer geringen Erhöhung des Luftwiderstandes, der dem Luftstrom im Gehäuse entgegenwirkt.

Weiterhin ist erfindungsgemäß vorgesehen, dass die mindestens eine Beleuchtungseinheit in Form einer Platine ausgestaltet ist, die mehrere in Reihen angeordnete Leuchtdioden in äquidistantem Abstand zueinander aufweist. Durch die Anordnung mehrerer Leuchtdioden wird erreicht, dass nahezu die gesamte Oberfläche der mindestens einen Luftreinigungseinheit mit Licht beaufschlagt wird und die Lichtverteilung im Gehäuse nahezu gleich ist.

Es muss stets eine gleichmäßige Bestrahlung der Oberfläche gewährleistet sein, um eine möglichst gute Reinigungswirkung zu erzielen.

Außerdem ist erfindungsgemäß vorgesehen, dass die Platine auf der Oberseite und auf der Unterseite mehrere Reihen von Leuchtdioden besitzt. Dadurch kann die Platine, wenn Sie mittig im Gehäuse angeordnet ist, beide Gehäusehälften beleuchten.

Schließlich ist erfindungsgemäß vorgesehen, dass im Gehäuse Luftleitelemente angeordnet sind, welche den Luftstrom auf den mindestens einen photokatalytisch wirksamen Oberflächenbereich der mindestens einen Luftreinigungseinheit ablenken. Zusätzlich entstehen dadurch gewollte Verwirbelungen der Luft. Damit wird eine besonders hohe Reinigungsleistung erzielt.

In einer vorteilhaften Ausgestaltung der Erfindung nach Patentanspruch 6 ist vorgesehen, dass die Luftleitelemente auf der Oberseite und der Unterseite der Platine angeordnet sind. Dies vereinfacht den Herstellungsprozess, die Anzahl der Einzelteile und den Zusammenbau der Vorrichtung.

In einer vorteilhaften Ausgestaltung der Erfindung nach Patentanspruch 7 ist vorgesehen, dass die Luftreinigungseinheit im oder beim Lufteinzug der Umluftklappe eines Kraftfahrzeuges angeordnet ist. Es wird in diesem Fall kein eigener Lüfter für die Luftreinigungseinheit benötigt, da der Luftfluss des Umluftbetriebs ausgenutzt und mitgenutzt werden kann.

In einer vorteilhaften Ausgestaltung der Erfindung nach Patentanspruch 8 ist vorgesehen, dass ein VOC Sensors zur bedarfsgerechten Steuerung der Luftreinigungseinheit in dieser angeordnet ist oder dass ein im Kraftfahrzeug bereits vorhandenen CO2 Sensors zur bedarfsgerechten Steuerung der Luftreinigungseinheit eingebunden ist.

Im Folgenden wird die erfindungsgemäße Luftreinigungssvorrichtung anhand konkreter Ausführungsbeispiele in den Figuren beschrieben. Die nachfolgende Beschreibung anhand der konkreten Ausführungsbeispiele stellt keine Limitierung der Erfindung auf eines dieser konkreten Ausführungsbeispiele dar.

In den Figuren zeigt:
- FIG 1: einen schematischen Aufbau einer Luftreinigungsvorrichtung;
- FIG 2: eine perspektivische Ansicht einer Luftreinigungsvorrichtung;
- FIG 3: eine Ansicht einer erfindungsgemäßen Luftreinigungsvorrichtung mit den weiteren relevanten Bestandteilen;
- FIG 4: eine erfindungsgemäße Luftreinigungssvorrichtung mit seitlich geöffnetem Gehäuse;
- FIG 5: eine perspektivische Darstellung einer erfindungsgemäßen Luftreinigungssvorrichtung mit seitlich geöffnetem Gehäuse;
- FIG 6: einen Blick in den Lufteinlasskanal der Luftreinigungsvorrichtung;
- FIG 7: einen Blick in den Luftausgangskanal der Luftreinigungssvorrichtung;
- FIG 8: eine Reinigungseinheit;
- FIG 9: eine Beleuchtungseinheit;

In den Figuren sind gleiche Teile und/oder Komponenten mit gleichen Bezugszeichen versehen. Diese Teile und/oder Komponenten entsprechen im Wesentlichen einander, solange nichts anderen angegeben ist.

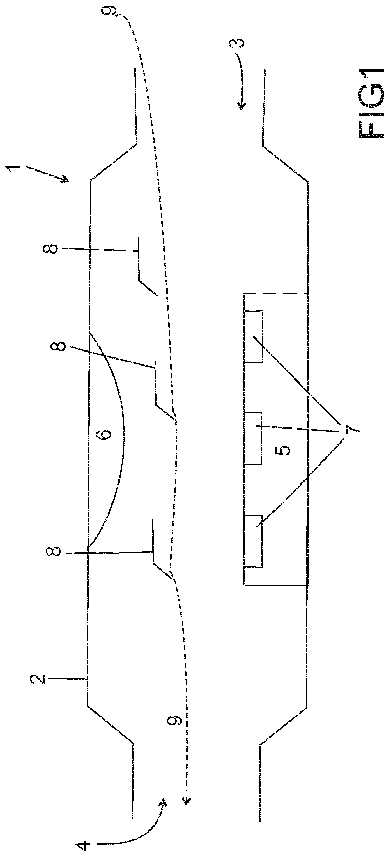

In FIG 1 ist ein Schnitt durch eine schematische Luftreinigungsvorrichtung 1 dargestellt. Die Luftreinigungsvorrichtung 1 besitzt ein Gehäuse 2. Die Form des Gehäuses 2 kann einen runden, ovalen, sechseckigen, n-eckigen oder, vorzugsweise, einen rechteckigen Querschnitt besitzen.

Als besonders vorteilhaft hat sich die Ausgestaltung des Querschnitts des Gehäuses 2 in einer rechteckigen Form ergeben. Das Gehäuse 2 ist quaderförmig.

Das Gehäuse 2 besteht vorzugsweise aus Kunststoff; in einer besonderen Ausgestaltung ist der gewählte Kunststoff ABS.

Das Gehäuse 2 weist an zwei sich gegenüberliegenden Enden eine Einlassöffnung in Form eines Lufteinlasskanals 3 und eine Auslassöffnung in Form eines Luftauslasskanals 4 für über den Lufteinlasskanal 3 dem Gehäuse 2 zugeführte Luft, auf.

Über den Lufteinlasskanal 3 wird zu reinigende Luft der Luftreinigungsvorrichtung 1 in deren Gehäuse 2 zugeführt; über den Luftauslasskanal 4 wird die in der Luftreinigungsvorrichtung 1 gereinigte Luft wieder aus dem Gehäuse 2 herausgeleitet.

Im Gehäuse 2 bildet sich ein Luftstrom 9. Der Luftstrom 9 durchläuft das Gehäuse 2 vom Lufteinlasskanal 3 zum Luftauslasskanal 4. Der Luftstrom ist im Gehäuse 2 gerichtet.

Im Gehäuse 2 ist eine Luftreinigungseinheit 5 angeordnet. Es können mehrere Luftreinigungseinheiten 5 im Gehäuse 2 angeordnet sein.

Die Luftreinigungseinheit 5 ist an einer der Innenwände des Gehäuses 2 angeordnet und ist mit der Innenwand form- oder kraftschlüssig verbindbar. Hierzu sind Klipse oder Halter vorgesehen, welche die Luftreinigungseinheit 5 an der Innenwand des Gehäuses 2 fixieren.

Auf der der Luftreinigungseinheit 5 gegenüberliegenden Innenwand des Gehäuses 2 ist eine Beleuchtungseinheit 6 angeordnet. Es können aber auch mehrere Beleuchtungseinheiten 6 im Gehäuse 2 angeordnet werden.

Die Beleuchtungseinheit 6 ist an der Innenwand des Gehäuses 2 so angeordnet, dass das von der Beleuchtungseinheit 6 emittierte Licht nahezu vollständig auf die Luftreinigungseinheit 5 gerichtet abgegeben wird.

In einer vorteilhaften Ausgestaltung der Erfindung sind vor der Beleuchtungseinheit 6 Linsen angeordnet, die das Licht der Beleuchtungseinheit 6 auf die Luftreinigungseinheit 5 fokussieren.

In einer weiteren Ausgestaltung der Erfindung sind Prismen oder Spiegel an der Beleuchtungseinheit 6 angeordnet, welche verhindern, dass das abgegebene Licht der Beleuchtungseinheit 6 nicht auf die Luftreinigungseinheit 5 trifft.

In einer weiteren vorteilhaften Ausgestaltung der Erfindung ist das Innere des Gehäuses 2 weiß lackiert oder mit einer lichtreflektierenden Oberflächenbeschichtung versehen.

Die Luftreinigungseinheit 5 besitzt mindestens einen photokatalytisch wirksamen Bereich 7. In der Ausgestaltung nach FIG 1 sind drei derartige Bereiche 7 vorhanden.

Die photokatalytisch wirksamen Bereiche 7 bestehen aus Titandioxid - TiO2 - oder sind mit Titanoxidionen - TiO2-Ionen - dotiert.

Die Beleuchtungseinrichtung 6 ist nunmehr auf die photokatalytisch wirksamen Bereiche 7 abgestimmt. Die Beleuchtungseinheit 6 gibt UV-Licht oder sichtbares Licht mit einer voreinstellbaren oder voreingestellten Wellenlänge ab. Die Photonen mit der entsprechenden Wellenlänge des UV-Lichts oder sichtbaren Lichts der Beleuchtungseinrichtung 6 lösen beim Auftreffen auf den photokatalytisch wirksamen Bereich 7 eine photochemischen Reaktion im Titanoxid aus, die dazu führt, dass Geruchsstoffpartikel und/oder Schadpartikel in der Luft umgewandelt bzw. zerstört werden. Die auf die photokatalytisch wirksamen Bereiche 7 auftreffenden Geruchsstoffpartikel und/oder Schadpartikel werden durch den photochemischen Prozess zerstört oder umgewandelt und damit wird die zugeführte Luft gereinigt.

Bei der Beleuchtungseinrichtung 6 handelt es sich um mindestens eine Leuchtdiode, vorzugsweise eine UV-Leuchtdiode. UV hat die Bedeutung von ultraviolett.

Erfindungsgemäß sind mehrere UV-Leuchtdioden angeordnet und bilden die Beleuchtungseinrichtung 6. Die Leuchtdioden sind zueinander in einer Reihe äquidistant zueinander angeordnet. Es sind dann mehrere Reihen von UV-Leuchtdioden parallel nebeneinander und in gleichem Abstand zueinander angeordnet.

Die Beleuchtungseinrichtung 6 wird über eine nicht in FIG 1 dargestellten Steuereinheit angesteuert.

Im Gehäuse 2 sind Luftleitelemente 8 angeordnet. Diese dienen dazu, den Luftstrom 9 im Gehäuse 2 auf die Luftreinigungseinheit 5 und die photokatalytisch wirksamen Bereiche 7 zu lenken, sodass ein möglichst großer Teil des Luftstroms 9 mit den Schadpartikeln und/oder Schadstoffpartikeln zu den photokatalytisch wirksamen Bereichen 7 gelangt, um dort photokatalytisch zu reagieren. Um eine möglichst gute Luftumströmung der photokatalytisch wirksamen Bereiche 7 der Luftreinigungseinheit 5 zu erreichen, und zugleich nicht die Lichtabgabe der Beleuchtungseinheit 6 auf die Luftreinigungseinheit 5 zu beeinträchtigen, sind die Luftleitelement 8 nicht oberhalb der photokatalytisch wirksamen Bereiche 7 der Luftreinigungseinheit 5 angeordnet, sondern vor und nach der Beleuchtungseinheit 6.

Durch die Reinigungseinheit 5 und deren potokatalytisch wirksamen Bereiche 7 wird, bei Auftreffen von UV-Licht, abgestrahlt von der Beleuchtungseinheit 6, entsprechend die durchströmende Luft gereinigt.

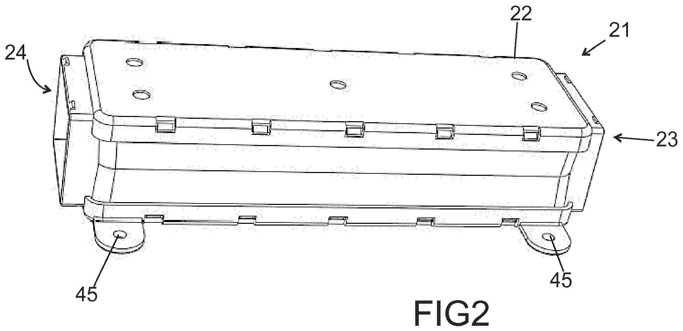

In FIG 2 ist ein Gehäuse 22 einer weiteren Ausgestaltung einer weiteren Luftreinigungsvorrichtung 21 dargestellt. Das Gehäuse 21 besteht aus mehreren Einzelteilen, die sich zu dem Gehäuse 22 zusammensetzen lassen. Das Gehäuse 22 ist quaderförmig ausgestaltet und weist zur einen Seite eine Verjüngung auf, die in den Lufteinlasskanal 23 übergeht. Auf der dem Lufteinlasskanal 23 gegenüberliegenden Seite des Gehäuses 22 ist eine weitere entsprechende Verjüngung vorgesehen, die die den Luftauslasskanal 24 bildet. Die zu reinigende Luft wird dem Kraftfahrzeuginnenraum entnommen, insbesondere aus diesem abgesaugt, und über den Lufteinlasskanal 23 dem Gehäuse 22 und damit der Luftreinigungsvorrichtung 21 zugeführt. Im Gehäuse 22 erfolgt dann die Reinigung der Luft, analog wie bei FIG 1 beschrieben, und die gereinigte Luft wird dann über den Luftauslasskanal 24 ausgeleitet und dann dem Fahrzeuginnenraum wieder zugeführt.

Im Weiteren sind am Gehäuse Aufnahmen 45 vorhanden, die fest mit dem Gehäuse 22 verbunden sind. Diese Aufnahmen 45 dienen dazu, die Luftreinigungsvorrichtung 21 in einem Kraftfahrzeug an eine vorgesehene Stelle befestigen zu können. Hierzu ist eine Klemmung oder eine Verschraubung vorgesehen.

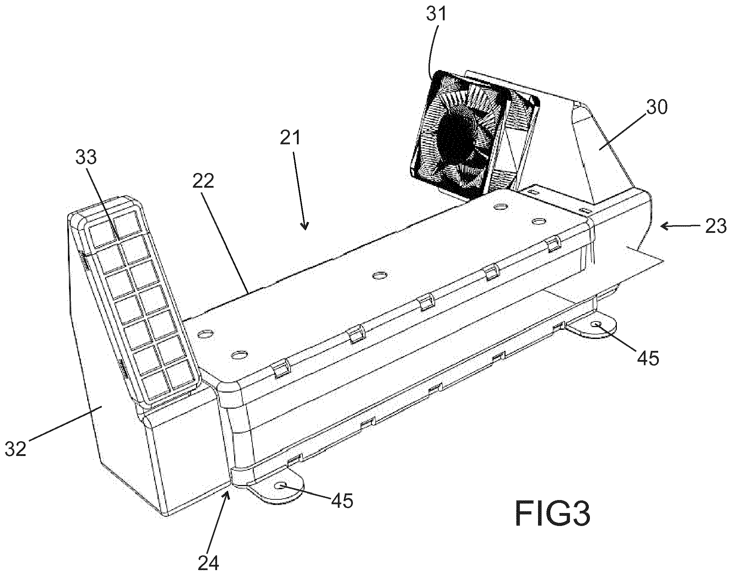

In FIG 3 ist die Luftreinigungsvorrichtung 21 mit dem Gehäuse 22 dargestellt. Es ist am Lufteinlasskanal 23 ein Luftzuführungskanal 30 angeordnet, an dessen Eingang ein Ventilator 31 angeordnet ist, der Luft aus dem Fahrzeuginnenraum ansaugt und durch den Luftzuführungskanal 30 und über den Lufteinlasskanal 23 in das Gehäuse 22 der Luftreinigungsvorrichtung 21 bläst, vorhanden. Der Luftzuführungskanal 30 ist auf die Verjüngung des Lufteinlasskanals 23 aufgesteckt und ist formschlüssig mit diesem verbunden.

In einer besonderen Ausgestaltung der Erfindung ist der Luftzuführungskanal 30 mittels einer Schnappvorrichtung mit dem Lufteinlasskanal 23 oder dem Gehäuse 22 verbindbar. Auf der Seite der Luftausgangsöffnung 24 ist ein Luftführungskanal 32 angeordnet, der die ausströmende Luft aus der Luftausgangsöffnung 24 aufnimmt, kanalisiert und entsprechend an den Innenraum eines Kraftfahrzeuges, aus dem mittels des Ventilators 31 die Luft abgesaugt worden ist, wieder zugeführt.

In einer weiteren vorteilhaften Ausgestaltung der Erfindung ist vorgesehen, dass in dem Luftführungskanal 32 mindestens eine Luftfiltereinheiten 33, wie beispielsweise ein HEPA-Filter oder ein anderer Luftfilter, angeordnet ist.

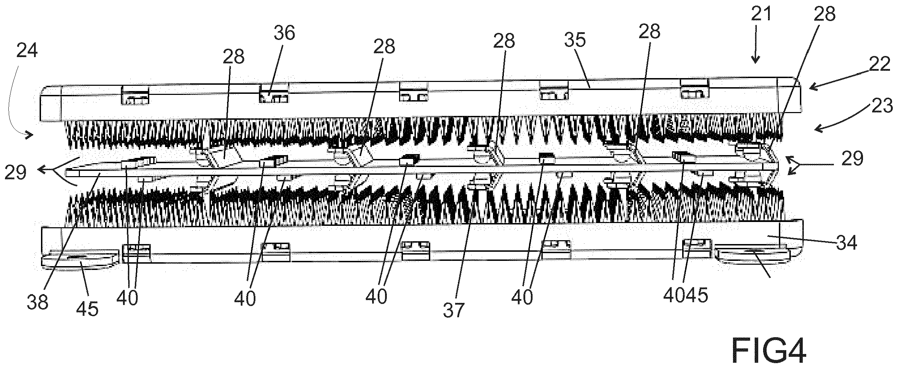

In FIG 4 ist das Gehäuse 22 der Luftreinigungsvorrichtung 21 seitlich geöffnet dargestellt. Es sind die relevanten Komponenten, die im Gehäuse 22 angeordnet ist, dargestellt.

Die zu reinigende Luft wird in Form eines Luftstromes 29 dem Gehäuse 22 über den Lufteinlasskanal 23 zugeführt und die gereinigte Luft in Form des Luftstromes 29 über den Luftauslasskanal 24 aus dem Gehäuse 22 ausgeleitet.

Das Gehäuse 22 besitzt einen Gehäuseboden 34 und einen Gehäusedeckel 35, die gemeinsam mit den nicht in FIG 4 dargestellten Gehäuseseitenteilen das Gehäuse 22 bilden. Am Gehäuseboden 34 sind die Aufnahmen 45 angeordnet. Seitlich außenseitig weisen sowohl der Gehäuseboden 34 als auch der Gehäusedeckel 35, mehrere Einbuchtungen 36 auf, in welche korrespondierende Nasen an den Gehäuseseitenteilen einschnappen und so den Gehäuseboden 34 und den Gehäusedeckel 35 über die Gehäuseseitenteile verbinden.

Auf der Innenseite des Gehäusedeckels 35 und der Innenseite des Gehäusebodens 34 ist jeweils eine Luftreinigungseinheit 25 angeordnet. Eine Luftreinigungseinheit 25 erstreckt sich nahezu über die gesamte Fläche des Gehäusebodens 34 bzw. des Gehäusedeckels 35. Jede Luftreinigungseinheit 25 ist form-und/oder kraftschlüssig mit dem Gehäuseboden 34 oder dem Gehäusedeckel 35 verbunden. Diese Verbindung erfolgt beispielsweise über eine Verklipsung, Verklebung oder Verschraubung.

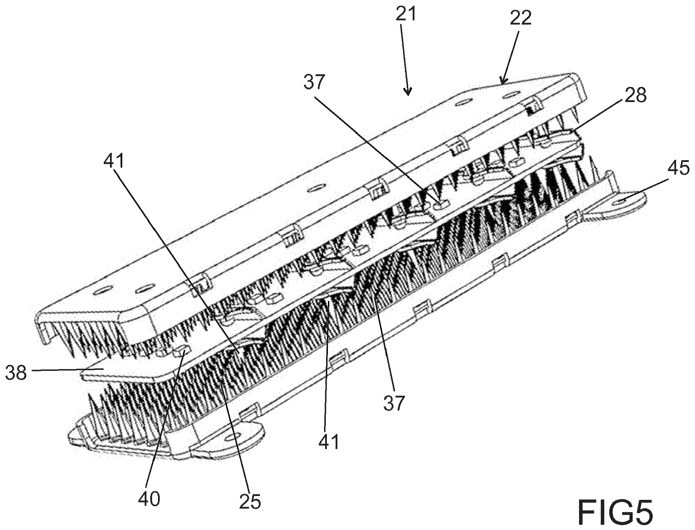

Eine jede der zwei Luftreinigungseinheiten 25, eine im Gehäusedeckel 35 und eine am Gehäuseboden 34, ist nunmehr in ihrer Formgestaltung, wie in FIG 5 dargestellt, optimiert, zum einen, um eine möglichst große Oberfläche aufzuweisen und zum anderen, um dem Luftstrom 29 im Gehäuse 22 strömungstechnisch geringstmöglich entgegenzuwirken. Damit wird der Druckverlust im Gehäuse 22 möglichst gering gehalten, zugleich aber der Luftstrom 29 an einer möglichst großen Oberfläche der beiden Luftreinigungseinheiten 25 vorbeigeführt und mit diesen in Berührung gebracht.

In der konkreten Ausführung gem. FIG 4 sind Pyramidenformen 37, mit quadratischer oder n-eckiger Grundfläche dargestellt. Diese sehen auch aus wie Zähne, die vom Gehäuseboden 34 nach oben ragen bzw. vom Gehäusedeckel 35 herabhängen. Diese Pyramidenformen 37 sind jeweils äquidistant zueinander angeordnet.

In einer erfindungsgemäßen Ausgestaltung der Erfindung ist vorgesehen, dass die Oberfläche der Luftreinigungseinheit 25, die mit Titandioxid - Ti02 - beschichtet oder teilbeschichtet ist bzw. mit Titandioxid-Ionen - TiO2-Ionen - dotiert ist, in Form einer kegel-, falten-, zylinder-, kegelstumpf-, pyramidenstumpf-, kugel- oder halbkugelgeometrischen Form, oder in igelförmig, ausgestaltet.

Die Luftreinigungseinheit 25 mit den Pyramidenformen 37 ist nunmehr mit Titanoxidionen - TIO2-Ionen - dotiert oder mit Titandioxid - TI02 - überzogen. Dies erfolgt vorrangig im Bereich der Pyramidenformen 37.

Gemäß der Erfindung ist vorgesehen, dass zumindest Teilbereiche der Luftreinigungseinheit 25 mit Titanoxiddionen dotiert oder mit Titanoxid teilbeschichtet oder vollständig beschichtet sind.

Durch die gewählte Pyramidenformen 37 ist es nunmehr möglich, dass der das Gehäuse 22 durchströmende Luftstrom 29 diese Pyramidenformen 37 der Luftreinigungseinheiten 25 umströmt und damit die Luftreinigungseinheit 25 eine möglichst große photokatalytisch wirksame Fläche besitzt.

Mittig im Gehäuse 22 ist eine Platine 38 angeordnet. Auf der Platine 38 sind nunmehr auf der Oberseite der Platine 38 und der Unterseite der Platine 38 Leuchtdiodenreihen 39 mit mehreren nebeneinander in einer Reihe, nahezu äquidistant zueinander, mehrere Leuchtdioden 40, angeordnet. Bei den Leuchtdioden 40 handelt es sich vorzugsweise um Leuchtdioden, die UV-Licht emittieren.

Die Leuchtdioden 40, die in den Leuchtdiodenreihen 39 angeordnet sind, bilden die Beleuchtungseinheit 26, die dazu dient, die photokatalytische Reaktion auszulösen. Durch das UV-Licht der Leuchtdioden 40, das in Richtung Pyramidenformen 37 der Luftreinigungseinheit 25 strahlt, wird der photokatalytische Prozess in Gang gesetzt.

Der vorbeiströmende Luftstrom 29 wird photokatalytisch gereinigt.

In der Ausgestaltung nach FIG 4 wird der Luftstrom 29 im Gehäuse 22 aufgeteilt. Um nun den Luftstrom 29 besonders gut mit den photokatalytisch wirksamen Bereichen der Luftreinigungseinheiten 25 in Kontakt zu bringen, sind auf der Platine 38 Luftleitelemente 28 angeordnet. Diese dienen dazu, den Luftstrom 29 auf die photokatalytisch wirksamen Bereiche zu lenken.

FIG 5 zeigt eine perspektivische Darstellung von FIG 4. Es ist die Ausgestaltung der Form der Luftreinigungseinheit 25 mit der Ausgestaltung der Pyramidenformen 37 deutlich erkennbar, ebenso die Anordnung der Leuchtdioden 40 in Leuchtdioden-Reihen 39 und der auf der Platine 38 angeordneten Luftleitelemente 28. Es sind weiterhin Stützen 41 vorhanden, welche von den am Gehäuseboden 34 oder am Gehäusedeckel 35 befestigten Luftreinigungseinheiten 25 in Richtung der Platine 38 abstehen. Diese Stützen sind am Gehäuseboden 34 oder am Gehäusedeckel 35 anordenbar; in dafür vorgesehenen Aussparungen und auf diesen Stützen 39 ist die Platine 38 fixierbar. Die Platine 38 ist im Gehäuse 22 in etwa mittig angeordnet und weist zum Gehäuseboden 34 und zum Gehäusedeckel 35 einen nahezu gleichen Abstand auf.

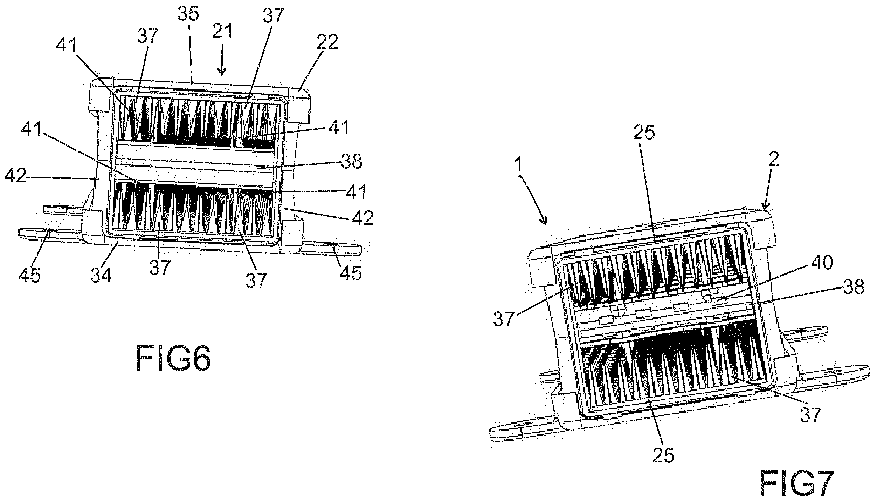

In FIG 6 ist eine Draufsicht auf den Luftzuführungskanal 23 dargestellt. Es sind die Stützen 41 ersichtlich, welche die Platine 38 im Gehäuse 22 fixieren. Die Gehäuseseitenteile 42 sind in Führungen im Gehäusedeckel 35 und Gehäuseboden 34 einsetzbar und werden über die Einbuchtungen 36 durch Einschnappen in selbigen fixiert.

Die Aufnahmen 45 dienen zur Befestigung der Luftreinungunsvorrichtung 1.

In FIG 7 ist eine Draufsicht auf den Luftaustrittskanal 24 dargestellt. Es sind wiederrum die Stützen 41 angeordnet, welche die Platine 38 mittig im Gehäuse 22 halten. Ebenso ist die Anordnung der Leuchtdioden 40 auf der Platine 38 dargestellt und die Formgebung der Luftreinigungseinheiten 25 und deren zur Reinigung der Luft und zur Luftwiderstandsoptimierung ausgestalteten Oberfläche mittels Pyramidenformen 37.

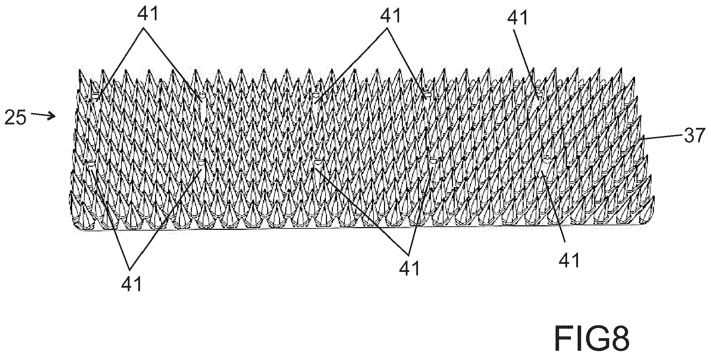

In FIG 8 ist nunmehr eine Luftreinigungseinheiten 25 mit der Oberflächengestaltung in Form von Pyramidenformen 37 dargestellt. Die photokatalytisch wirksamen Bereiche der Pyramidenformen 37 werden dadurch erzeugt, dass die Oberfläche der Luftreinigungseinheit zunächst aus Kunststoff mit einem bekannten und gängigen Herstellungsverfahren erzeugt wird und anschließend die Oberfläche mit einem Titanoxid - TiO2 - in Pulverform beschichtet wird. Hierzu kann das Resin-Verfahren zum Einsatz kommen. Es besteht aber auch die Möglichkeit die Oberfläche über ein SOL-Gel Verfahren zu beschichten. Des Weiteren kann dotiertes TiO2 zum Einsatz kommen, das einerseits eine höhere Effizienz bei der Bestrahlung mit UV aufweist oder andererseits durch Einbringung von Molekülen so verändert wurde, dass bereits bei sichtbarem Licht eine hohe Aktivität besteht. Auch kann der Katalysator bereits im Kunststoff enthalten (TiO2 dotierte Kunststoff) sein und freigebrannt werden. Es besteht die Möglichkeit des Aufdampfens oder Pulverlackierens. Zudem besteht die Möglichkeit andere Photokatalysatoren wie LiNbO3 zu nutzen.

Durch die konkrete Ausgestaltung der Oberfläche mit Pyramidenformen 37 wird bei einer Grundfläche der Luftreinigungseinheit 25 von 117 Quadratzentimetern eine Oberfläche von ca. 521 Quadratzentimetern geschaffen, d.h. die Oberfläche der Luftreinigungseinheit 25 ist fast um den Faktor Fünf größer als die Grundfläche.

Dadurch kann durch die Beschichtung mit Titandioxid - TIO2 - eine große Fläche mit photokatalytisch wirksamen Bereichen geschaffen werden.

Es sind die Stützen 41 als Bestandteil der Luftreinigungseinheit 25 integriert. Bei Beschichtung oder Dotierung mit Titandioxid - TIO2 - wirken die Stützen 41 ebenfalls als photokatalytisch wirksame Bereiche.

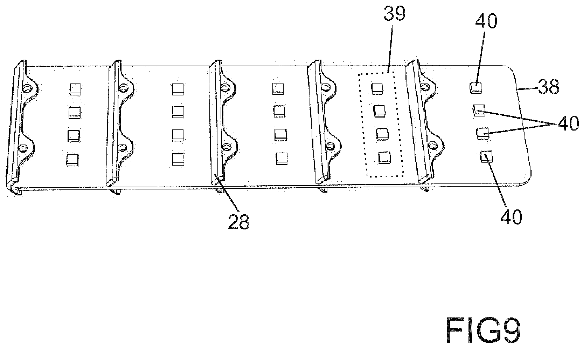

In FIG 9 ist die Platine 38 in einer Draufsicht dargestellt. Die nicht sichtbare Unterseite ist analog gestaltet. Die Platine 38 besitzt jeweils in Reihen 39 angeordnet vier Leuchtdioden 40. Es sind insgesamt fünf Leuchtdiodenreihen 39 vorhanden, jeweils auf der Ober- und Unterseite der Platine 38. Im Weiteren sind die Luftleitelemente 28 dargestellt.

Bei den Leuchtdioden 40 handelt es sich um Leuchtdioden, die ultraviolettes Licht emittieren, das vorzugsweise auf das Titandioxid und dessen dadurch erzeugbaren photokatalytischen Wirkung abgestimmt ist, und eine Wellenlänge im Bereich des sichtbaren Lichts zwischen 400 und 500 Nanometer besitzt, vorzugsweise im Bereich um die 450 Nanometer. Bei Einsatz von dotiertem TiO2 sollte UV-A Licht zum Einsatz kommen, vorzugsweise mit 367 nm Wellenlänge.

### Bezugszeichenliste

- 1, 21: Luftreinigungsvorrichtung
- 2, 22: Gehäuse
- 3, 23: Lufteinlasskanal
- 4, 24: Luftauslasskanal
- 5, 25: Luftreinigungseinheit
- 6, 26: Beleuchtungseinheit
- 7, 27: photokatalytisch wirksamer Bereich der Luftreinigungseinheit
- 8, 28: Luftleitelemente
- 9, 29: Luftstrom
- 30: Luftzuführungskanal
- 31: Ventilator
- 32: Luftführungskanal
- 33: Luftfiltereinheit
- 34: Gehäuseboden
- 35: Gehäusedeckel
- 36: Einbuchtungen
- 37: Pyramidenformen
- 38: Platine
- 39: Leuchtdioden-Reihen
- 40: Leuchtdioden
- 41: Stützen
- 42: Gehäuseseitenteile

- 45: Aufnahmen

## Patentansprüche

1. Luftreinigungseinheit (1, 21), bestehend aus einem Gehäuse (2, 22), das mindestens eine Eingangsöffnung (3, 23) zur Zuführung eines Luftstroms (9, 29) und mindestens eine Ausgangsöffnung (4 24) zur Ausleitung des über die Eingangsöffnung (3, 23) zugeführten Luftstroms (9, 29) besitzt, wobei im Gehäuse (2, 22) mindestens eine Luftreinigungseinheit (5, 25) und mindestens eine Beleuchtungseinheit (6, 26) angeordnet sind, wobei die mindestens eine Luftreinigungseinheit (5, 25) und die mindestens eine Beleuchtungseinheit (6, 26) einander gegenüberliegend im Gehäuse (2, 22) angeordnet sind, die mindestens eine Luftreinigungseinheit (5, 25), mindestens einen photokatalytisch wirksamen Oberflächenbereich (7, 27) besitzt und der Luftstrom (9, 29) im Gehäuse (2, 22) zumindest partiell entlang des mindestens einen photokatalytisch wirksamen Oberflächenbereichs (7, 27) der mindestens einen Luftreinigungseinheit (5, 25) geführt ist, wobei die der mindestens eine photokatalytisch wirksame Oberflächenbereich (7, 27) der mindestens einen Luftreinigungseinheit (5, 25) von der mindestens einen Beleuchtungseinheit (6, 26) mit Licht anstrahlbar ist und der mindestens eine photokatalytisch wirksame Oberflächenbereich (7) zumindest teilweise mit Titandioxid - TiO2 - beschichtet oder mit Titandioxidionen - TiO2-Ionen - dotiert ist,**dadurch gekennzeichnet, dass** die mindestens eine Luftreinigungseinheit (5, 25) aus zwei Luftreinigungseinheiten (5, 25) besteht, die einander gegenüberliegend an den Innenwänden des Gehäuses (2, 22) angeordnet ist , wobeidie mindestens eine Beleuchtungseinheit (6, 26) im Gehäuse (2, 22) in der Mitte des Gehäuses (2, 22) dieses in einer Ebene durchlaufend, angeordnet ist, sodass das von der mindestens eine Beleuchtungseinheit (6, 26) emittierte Licht nahezu vollständig auf die zwei Luftreinigungseinheiten (5, 25) auftrifft, wobei die zwei Luftreinigungseinheiten (5, 25) auf dem katalytisch wirksamen Oberflächenbereich (7, 27) aufweisenden Seite mit einer kegel-, falten-, zylinder-, kegelstumpf-, pyramidenstumpf-, kugel- oder halbkugelgeometrischen Form, oder igelförmig, ausgestaltet sind und die mindestens eine Beleuchtungseinheit (6, 26) in Form einer Platine (38) ausgestaltet ist, die mehrere in Reihen (39) angeordnete Leuchtdioden (40) in äquidistantem Abstand zueinander aufweist wobei die Platine (38) auf der Oberseite und auf der Unterseite mehrere Reihen (39) von Leuchtdioden (40) besitzt und wobei im Gehäuse (2, 22) Luftleitelemente (8, 28) angeordnet sind, welche den Luftstrom (9, 29) auf den mindestens einen photokatalytisch wirksamen Oberflächenbereich (7, 27) der mindestens eine Luftreinigungseinheit (5, 25) ablenken.

2. Luftreinigungseinheit (1, 21) Patentanspruch 1, **dadurch gekennzeichnet, dass** vor der mindestens einen Beleuchtungseinheit (6, 26) Linsen angeordnet sind, die das von der mindestens einen Beleuchtungseinheit (6, 26) emittierte Licht auf die mindestens einen Luftreinigungseinheit (5, 25) fokussieren oder hinter und/oder neben der mindestens einen Beleuchtungseinheit (6, 26) Lichtreflektoren oder Licht reflektierende Bereiche angeordnet sind, welche das von der mindestens einen Beleuchtungseinheit (6, 26) emittierte Licht in Richtung der mindestens einen Luftreinigungseinheit (5, 25) reflektiert.

3. Luftreinigungseinheit (1, 21) nach Patentanspruch 1 oder 2, **dadurch gekennzeichnet, dass** die Innenwände des Gehäuses (2, 22) weiß lackiert oder mit einem lichtreflektierenden Material beschichtet sind.

4. Luftreinigungseinheit (1, 21) nach einem der vorangehenden Patentansprüche, **dadurch gekennzeichnet, dass** die mindestens einen Beleuchtungseinheit (6, 26) aus mindestens einer Leuchtdiode (40) besteht, die UV-Licht emittiert.

5. Luftreinigungseinheit (1, 21) nach einem der vorangehenden Patentansprüche, **dadurch gekennzeichnet, dass** die mindestens eine Luftreinigungseinheit (25) sich nahezu über die gesamte Fläche des Gehäusebodens (34) und/oder des Gehäusedeckels 35 des Gehäuses (22) erstreckt und am Gehäuseboden (34) und/oder Gehäusedeckel (35) fixierbar ist.

6. Luftreinigungseinheit (1, 21) nach Patentanspruch 1, **dadurch gekennzeichnet, dass** die Luftleitelemente (28) auf der Oberseite und der Unterseite der Platine (38) angeordnet sind.

7. Luftreinigungseinheit (1, 21) nach einem der vorangehenden Patentansprüche, **dadurch gekennzeichnet, dass** die Luftreinigungseinheit (1, 21) im oder beim Lufteinzug der Umluftklappe eines Kraftfahrzeuges angeordnet ist.

8. Luftreinigungseinheit (1, 21) nach einem der vorangehenden Patentansprüche, **dadurch gekennzeichnet, dass** ein VOC Sensor zur bedarfsgerechten Steuerung der Luftreinigungseinheit in dieser angeordnet ist oder dass ein im Kraftfahrzeug bereits vorhandener CO2-Sensor zur bedarfsgerechten Steuerung der Luftreinigungseinheit eingebunden ist.

## Claims

1. Air purification device (1, 21), consisting of a housing (2, 22) having at least one inlet opening (2, 23) for supply of an air flow (9, 29) and at least one outlet opening (4, 24) for discharge of the air flow (9, 29) supplied by way of the inlet opening (3, 23), wherein at least one air purifying unit (5, 25) and at least one lighting unit (6, 26) are arranged in the housing (2, 22), wherein the at least one air purifying unit (5, 25) and the at least one lighting unit (6, 26) are arranged opposite one another in the housing (2, 22), the at least one air purifying unit (5, 25) has at least one photocatalytically active surface region (7, 27) and the air flow (9, 29) is guided in the housing (2, 22) at least partially along the at least one photocatalytically active surface region (7, 27) of the at least one air purifying unit (5, 25), wherein the at least one photocatalytically active surface region (7, 27) of the at least one air purifying unit (5, 25) can be irradiated with light by the at least one lighting unit (6, 26) and the at least one photocatalytically active surface region (7) is coated at least partly with titanium dioxide (TiO₂) or doped with titanium dioxide ions (TiO₂ ions), **characterised in that** the at least one air purifying unit (5, 25) consists of two air purifying units (5, 25) arranged opposite one another at the inner walls of the housing (2, 22), wherein the at least one lighting unit (6, 26) is arranged in the housing (2, 22) in the centre of the housing (2, 22) to traverse this in a plane so that the light emitted by the at least one lighting unit (6, 26) is incident almost in its entirety on the two air purifying units (5, 25), wherein the two air purifying units (25) are shaped on the side having the catalytically active surface region (7, 27) with a conical, pleated, cylindrical, frusto-conical, frusto-pyramidal, spherical or hemispherical geometric form or are spiny and the at least one lighting unit (6, 26) has the form of a circuitboard (38) having a plurality of light-emitting diodes (40) arranged in rows (39) and at an equidistant spacing from one another, wherein the circuitboard (38) has a plurality of rows (39) of light-emitting diodes (40) on the upper side and on the lower side, and wherein air guide elements (8, 28) which deflect the air flow (9, 29) onto the at least one photocatalytically active surface region (7, 27) of the at least one air purifying unit (5, 25) are arranged in the housing (2, 22).

2. Air purification device (1, 21) according to claim 1, **characterised in that** lenses which focus the light emitted by the at least one lighting unit (6, 26) on the at least one air purifying unit (5, 25) are arranged in front of the at least one lighting unit (6, 26) or that light reflectors or light-reflecting regions which reflect the light emitted by the at least one lighting unit (6, 26) in the direction of the at least one air purifying unit (5, 25) are arranged behind and/or adjacent to the at least one lighting unit (6, 26).

3. Air purification device (1, 21) according to claims 1 or 2, **characterised in that** the inner walls of the housing (2, 22) are painted white and/or coated with a light-reflecting material.

4. Air purification device (1, 21) according to any one of the preceding claims, **characterised in that** the at least one lighting unit (6, 26) consists of at least one light-emitting diode (40) which emits ultraviolet light.

5. Air purification device (1, 21) according to any one of the preceding claims, **characterised in that** the at least one air purifying unit (25) extends over almost the entire area of the housing base (34) and/or the housing cover (35) of the housing (22) and is fixable to the housing base (34) and/or housing cover (35).

6. Air purification device (1, 21) according to claim 1, **characterised in that** the air guide elements (28) are arranged on the upper side and lower side of the circuitboard (38).

7. Air purification device (1, 21) according to any one of the preceding claims, **characterised in that** the air purification device (1, 21) is arranged in or at the air intake of the air circulation flap of a motor vehicle.

8. Air purification device (1, 21) according to any one of the preceding claims, **characterised in that** a volatile organic compound sensor for need-based control of the air purification device is arranged therein or a CO₂ sensor already present in the motor vehicle is incorporated for need-based control of the air purification device.

## Revendications

1. Unité de purification d'air (1, 21), constituée d'un boîtier (2, 22), qui possède au moins une ouverture d'entrée (3, 23) servant à amener un flux d'air (9, 29) et au moins une ouverture de sortie (4, 24) servant à évacuer le flux d'air (9, 29) amené par l'intermédiaire de l'ouverture d'entrée (3, 23), dans laquelle au moins une unité de purification d'air (5, 25) et au moins une unité d'éclairage (6, 26) sont disposées dans le boîtier (2, 22), dans laquelle l'au moins une unité de purification d'air (5, 25) et l'au moins une unité d'éclairage (6, 26) sont disposées face à face dans le boîtier (2, 22), l'au moins une unité de purification d'air (5, 25) possède au moins une zone de surface active photocatalytiquement (7, 27), et le flux d'air (9, 29) est guidé dans le boîtier (2, 22) au moins de manière partielle le long de l'au moins une zone de surface active photocatalytiquement (7, 27) de l'au moins une unité de purification d'air (5, 25), dans laquelle l'au moins une zone de surface active photocatalytiquement (7, 27) de l'au moins une unité de purification d'air (5, 25) peut être irradiée de lumière par l'au moins une unité d'éclairage (6, 26) et l'au moins une zone de surface (7) active photocatalytiquement est revêtue au moins en partie d'oxyde de titane - Ti02 - ou est dopée avec des ions d'oxyde de titane - ions Ti02, **caractérisée en ce que** l'au moins une unité de purification d'air (5, 25) est constituée de deux unités de purification d'air (5, 25), qui sont disposées face à face au niveau des parois intérieures du boîtier (2, 22), dans laquelle l'au moins une unité d'éclairage (6, 26) est disposée dans le boîtier (2, 22) au centre du boîtier (2, 22) en traversant celui-ci dans un plan de sorte que la lumière émise par l'au moins une unité d'éclairage (6, 26) arrive quasiment en totalité sur les deux unités de purification d'air (5, 25), dans laquelle les deux unités de purification d'air (5, 25) sont configurées sur le côté présentant la zone de surface (7, 27) active catalytiquement avec une forme géométrique de cône, de plis, de cylindre, de cône tronqué, de tronc de pyramide, de bille ou de demi-bille ou en forme de hérisson et l'au moins une unité d'éclairage (6, 26) est configurée sous la forme d'une platine (38), qui présente plusieurs diodes électroluminescentes (40) disposées en rangées (39) à distance équidistante les unes des autres, dans laquelle la platine (38) possède sur le côté supérieur et sur le côté inférieur plusieurs rangées (39) de diodes électroluminescentes (40) et dans laquelle dans le boîtier (2, 22) sont disposés des éléments de conduite d'air (8, 28), lesquels dévient le flux d'air (9, 29) sur l'au moins une zone de surface active photocatalytiquement (7, 27) de l'au moins une unité de purification d'air (5, 25).

2. Unité de purification d'air (1, 21) selon la revendication 1, **caractérisée en ce que** devant l'au moins une unité d'éclairage (6, 26) sont disposées des lentilles, qui concentrent la lumière émise par l'au moins une unité d'éclairage (6, 26) sur l'au moins une unité de purification d'air (5, 25) ou derrière et/ou à côté de l'au moins une unité d'éclairage (6, 26) sont disposés des réflecteurs de lumière ou des zones de réflexion de lumière, lesquels réfléchissent la lumière émise par l'au moins une unité d'éclairage (6, 26) en direction de l'au moins une unité de purification d'air (5, 25).

3. Unité de purification d'air (1, 21) selon la revendication 1 ou 2, **caractérisée en ce que** les parois intérieures du boîtier (2, 22) sont peintes en blanc ou sont revêtues d'un matériau réfléchissant la lumière.

4. Unité de purification d'air (1, 21) selon l'une quelconque des revendications précédentes, **caractérisée en ce que** l'au moins une unité d'éclairage (6, 26) est constituée d'au moins une diode électroluminescente (40), qui émet de la lumière UV.

5. Unité de purification d'air (1, 21) selon l'une quelconque des revendications précédentes, **caractérisée en ce que** l'au moins une unité de purification d'air (25) s'étend quasiment sur toute la surface du fond de boîtier (34) et/ou du couvercle de boîtier (35) du boîtier (22) et peut être fixée au niveau du fond de boîtier (34) et/ou du couvercle de boîtier (35).

6. Unité de purification d'air (1, 21) selon la revendication 1, **caractérisée en ce que** les éléments de conduite d'air (28) sont disposés sur le côté supérieur et sur le côté inférieur de la platine (38).

7. Unité de purification d'air (1, 21) selon l'une quelconque des revendications précédentes, **caractérisée en ce que** l'unité de purification d'air (1, 21) est disposée dans ou à proximité du système d'admission d'air du volet de circulation d'air d'un véhicule automobile.

8. Unité de purification d'air (1, 21) selon l'une quelconque des revendications précédentes, **caractérisée en ce qu'**un capteur COV aux fins de la commande conformément aux besoins de l'unité de purification d'air est disposé dans celle-ci ou qu'un capteur de CO2 déjà présent dans le véhicule automobile est rattaché aux fins de la commande conformément aux besoins de l'unité de purification d'air.
